# EUROPEAN PATENT APPLICATION

(11) **EP 4 360 679 A1**
(43) Date of publication of application: **01.05.2024**
(21) Application number: 22306634.1
(22) Date of filing: 28.10.2022
(51) Int. Cl.: A61M 5/32

(54) **A SYRINGE ASSEMBLY FOR USE IN MEDICATION DELIVERY**

(71) Applicant: Becton, Dickinson and Company, Franklin Lakes, NJ 07417-1880 (US)
(72) Inventor: CHANSAVANG, Albert, 38000 Grenoble (FR); MILLS, Freddy, 38000 Grenoble (FR)
(74) Representative: Germain Maureau

(57) **Abstract**

The syringe assembly for use in medication delivery comprises:
- a syringe barrel (12) having a needle (11) at a distal end (122) of the barrel (12);
- a tubular body (2) defining an enclosure, said barrel (12) extending at least partially within said enclosure;
- a safety shield (3) for the needle (11), longitudinally movable with respect to the tubular body (2) between a retracted position and an activated position;
- a spring (4) urging said safety shield (3) towards said activated position;
- a first locking mechanism (31) for locking the safety shield (3) in the retracted position, wherein the first locking mechanism (31) is manually releasable by a user so as to let the safety shield (3) reach the activated position.
- the tubular body (2) including at least one longitudinal slot (21), and the safety shield (3) including at least radial pin (30) engaging the longitudinal slot (21) such that the radial pin (30) moves from a proximal part (211) of the longitudinal slot (12) to a distal part (212) of the longitudinal slot (21) when the safety shield (3) moves from the retracted position towards the activated position;
- the first locking mechanism (31) being configured to hold the radial pin (30) at the proximal part (211) of the longitudinal slot (21).

## Description

### FIELD OF THE INVENTION

The present invention relates to a needle shielding system, and precisely to a syringe assembly for use in medication delivery, comprising a shield for enclosing a syringe needle after drug delivery completion.

### BACKGROUND OF THE INVENTION

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to the safety device or syringe of the invention, and the "proximal direction" is to be understood as meaning the opposite direction to said direction of injection, that is to say the direction towards the user's hand.

Pre-fillable or prefilled syringes, usually comprise a hollow tubular body or barrel forming a container for a medical product. This tubular body comprises a distal end in the form of a longitudinal tip defining an axial passageway through which the medical product is expelled from the container. The distal end is equipped with a needle for injection of the medical product into an injection site.

It is of great importance that the patients and users are protected from any risk of needle stick injuries, particularly between the moment that injection is finished and the discarding of the drug delivery device.

Needle shielding devices can be of the so called active type where a shielding device is activated by a user after injection. An example of shielding system is Becton Dickinson Preventis^{™} needle shielding system which enables one-handed, controlled activation that automatically shields the needle. After the injection, a further pressure on the piston rod triggers the shielding as disclosed for example by EP1397170.

This solution is very satisfactory, but it could be desirable that the activation force was completely independent from the injection movement and could be adjusted for usability purpose for instance to allow the shielding even after a partial injection which could be required in some cases to take into account the age or the weight of a patient.

### SUMMARY OF THE INVENTION

In a first aspect, the invention concerns a syringe assembly for use in medication delivery comprising:
- a syringe barrel having a needle at a distal end of the barrel;
- a tubular body defining an enclosure, said barrel extending at least partially within said enclosure;
- a safety shield for the needle, longitudinally movable with respect to the tubular body between a retracted position and an activated position;
- a spring urging said safety shield towards said activated position;
- a first locking mechanism for locking the safety shield in the retracted position, wherein the first locking mechanism is manually releasable by a user so as to let the safety shield reach the activated position.
- the tubular body including at least one longitudinal slot, and the safety shield including at least radial pin engaging the longitudinal slot such that the radial pin moves from a proximal part of the longitudinal slot to a distal part of the longitudinal slot when the safety shield moves from the retracted position towards the activated position;
- the first locking mechanism being configured to hold the radial pin at the proximal part of the longitudinal slot.

The syringe assembly can comprise a second locking mechanism for permanently and non-reversingly locking the safety shield in the activated position after the first locking mechanism is released.

The second locking mechanism can be configured to receive and then hold the radial pin at the distal part of the longitudinal slot.

The second locking mechanism can comprise a locking leg which is forcedly deflected by the radial pin when the safety shield moves from the retracted position towards the activated position.

The pin can radially protrude from the tubular body when it is at the proximal part of the longitudinal slot, and that the pin does not radially protrudes from the tubular body when it is at the distal part of the longitudinal slot.

The longitudinal slot can be provided with a rim enclosing the distal part of the longitudinal slot but not the proximal part of the longitudinal slot.

The locking leg can include at its distal end a cut configured and sized to receive the radial pin.

The distal part of the longitudinal slot can include a curved portion where the radial pin sits when the safety shield is its activated position.

The needle can be covered by the safety shield in the activated position and the needle is not covered by the safety shield in the retracted position.

The first locking mechanism can be a bayonet mount.

The syringe assembly can comprise a prefilled syringe comprising said barrel, said needle, a stopper and a plunger rod.

In a second aspect, the invention concerns a shielding system for a syringe for use in medication delivery comprising a barrel and a needle at a distal end of the barrel, said shielding system comprising:
- a tubular body defining an enclosure, said barrel extending at least partially within said enclosure;
- a safety shield for the needle, longitudinally movable with respect to the tubular body between a retracted position and an activated position;
- a spring urging said safety shield towards said activated position;
- a first locking mechanism for locking the safety shield in the retracted position, wherein the first locking mechanism is manually releasable by a user so as to let the safety shield reach the activated position;
- the tubular body including a longitudinal slot, and the safety shield including a radial pin engaging the longitudinal slot such that the radial pin moves from a proximal part of the longitudinal slot to a distal part of the longitudinal slot when the safety shield moves from the retracted position towards the activated position;
- the first locking mechanism being configured to hold the radial pin at the proximal part of the longitudinal slot.

In a third aspect, the invention concerns a method for operating a syringe assembly or a shielding system, comprising a step of triggering release of the radial pin by the first locking mechanism so that the spring moves the safety shield from the retracted position towards the activated position.

### BRIEF DESCRIPTION OF THE DRAWINGS

The invention and the advantages arising therefrom will clearly emerge from the detailed description that is given below with reference to the appended drawings as follows:
FIG. 1 is a top perspective view of an embodiment of a syringe assembly according to the present invention, with a needle safety shield in a retracted position;
FIG. 2 is a top perspective view of the syringe assembly shown in FIG. 1, with the needle safety shield in an activated position;
FIG. 3 is a view of a first locking mechanism of the syringe assembly (when in the state of FIG. 1);
FIG. 4 is a view of a second mechanism of the syringe assembly (when in the state of FIG. 2).

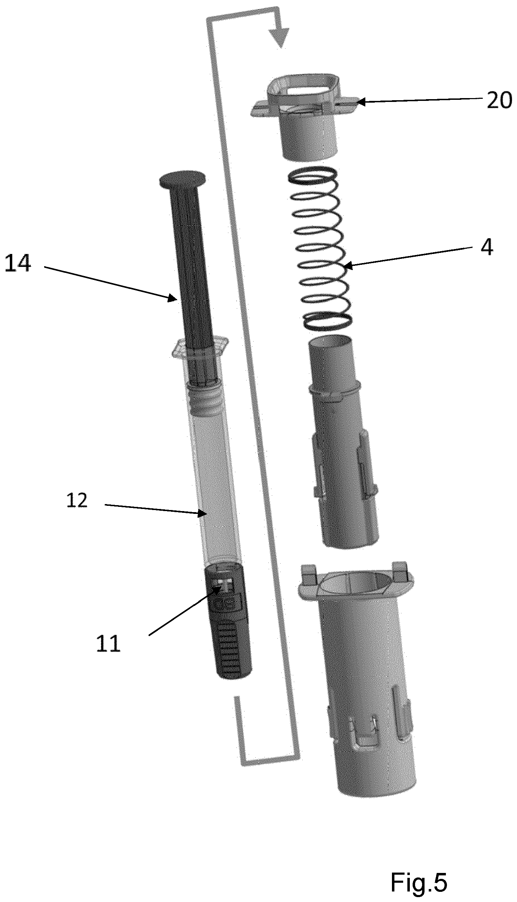

FIG. 5 is an exploded view of the syringe assembly of FIG.1.

### DETAILED DESCRIPTION OF EXEMPLARY EMBODIMENTS

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as distal, proximal, up, down, bottom, and top are relative, and are to aid illustration, but are not limiting. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure.

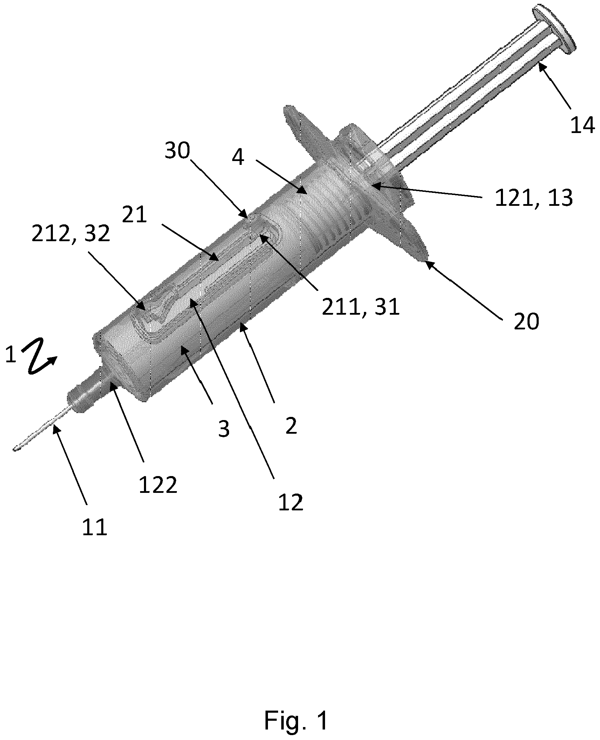

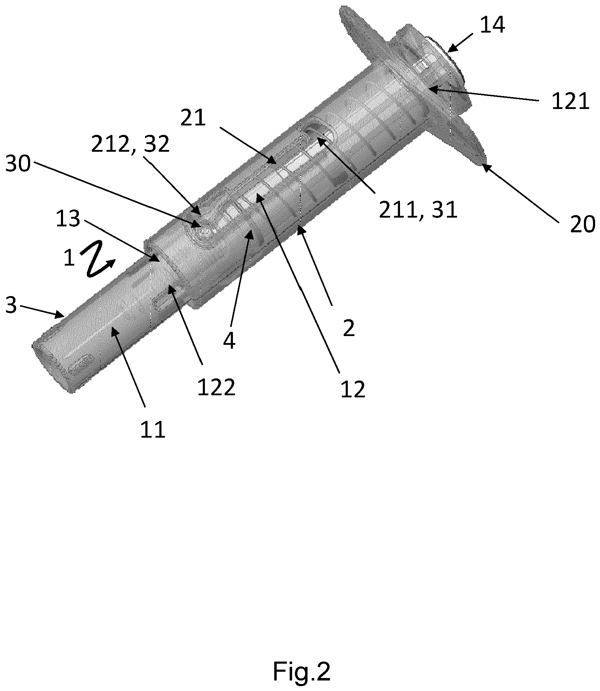

Figures 1 and 2 illustrate perspective views of a syringe assembly 10 in accordance with an embodiment of the present invention, respectively in a first state and a second state.

Said syringe assembly 10 is for use in medication delivery (in particular hypodermic injection) and preferably comprises a complete syringe 1 (in particular a pre-fillable syringe, which typically contains a single dose of medication such as such as 0.3 ml, 0.5 ml or 1.0 ml), coupled to a tubular body 2 which enables the shielding function. Syringes are typically comprised of a barrel 12, having a proximal end 121 and a distal end 122 with respect to said longitudinal axis; a needle 11 (or other piercing or connecting element such as a cannula) secured to a distal end of the barrel 12; a stopper 13 slidably positioned within the barrel 12; and a plunger rod 14 engageable with the stopper 13. The barrel 12 defines an inner fluid chamber. Forward movement (along said longitudinal axis) of the plunger rod 14 moves the stopper 13 from the proximal end 121 towards the distal end 122 (compare figure 1 and 2) which expels fluid from the barrel 12 through the needle 11. Rearward movement in the proximal direction of the plunger rod 14 will draw fluid into the barrel 12. Glass barrels are commonly used in prefillable or prefilled syringes, but plastic barrel are also known.

The syringe barrel 12 is coupled to a tubular body 2, also referred to as "holder". The tubular body 2 is preferably an elongate, generally cylindrical piece, in particular made of plastic (preferably a semi-rigid plastic material such as polypropylene), which defines a generally cylindrical enclosure which advantageously shares the same longitudinal axis with the barrel 12. The tubular body 2 has proximal and distal open ends which provide access to the enclosure. A finger flange 20 extends radially outwardly from the tubular body 2 near the proximal open end thereof. The finger flange 20 and tubular body 2 are designed for easy handling as an injection is made so that an injection can be carried out using a single hand. The syringe assembly also comprises a safety shield 3 for the needle 11, longitudinally movable with respect to the tubular body 2 - and consequently movable with respect to the barrel 12 - between a retracted position and an activated position, and a spring 4 configured for moving the shield 3. Preferably, the shield 3 is movable according to the same longitudinal axis as that of the barrel 12 and/or the tubular body 2. Like the tubular body 2, the shield 3 also has preferably an elongate, generally cylindrical piece, in particular made of plastic such as for example a semi-rigid plastic material such as polypropylene. The spring 4 is preferably a metal coil spring, but other energy releasing element can be contemplated, such as deformable blades. The length of the shield 3 is advantageously at least the length of the needle 11.

The syringe barrel 12 extends at least partially, and preferably substantially entirely, within the enclosure of the tubular body 2. The barrel 12 can be slidably engaged within the tubular body 2, and thus the syringe barrel 12 may include a radially outwardly extending flange for coupling onto an existing pre-fillable or prefilled syringe to the tubular body 2. In other embodiments, the tubular body 2 can be permanently secured on the barrel 12.

In any case, the needle safety shield 3 and the spring 4 can be placed inside the tubular body 2. An annular clearance is preferably kept in the enclosure between the tubular body 2 and the barrel 12 for housing the shield 3 and the spring 4. The safety shield 3 and the spring 4 are thus configured and sized in diameter to be positioned within the tubular body 2, and to fit over the barrel 12.

In some embodiments, the needle 11 is covered by the safety shield 3 in the activated position and the needle 11 is un-covered by the safety shield 3 in the retracted position. By "covering" the needle 11 is meant enclosing the needle tip such that it is not exposed, preventing any risk of injury.

In other words, in the retracted position the shield 3 is partially, and preferably entirely, engaged within the enclosure of the tubular body 2, such that the needle 11 is exposed for use, while in the activated position (or "extended" position) the shield 3 axially protrudes from the tubular body 2 toward the distal end, hence sheathing the needle 11.

We refer to as "first state" the state of the present syringe assembly wherein the shield is in the retracted state (see figure 1), and we refer to as "second state" the state of the present syringe assembly wherein the shield is in the activated state (see figure 2).

The spring 4 is urging said safety shield 3 towards said activated position. To this end, the tubular body 2 can include an end shoulder (close to its proximal open end) and in the retracted position, the spring 4 is compressed between the shield 3 and said end shoulder. Therefore, the spring 4 pushes the shield 3 in the longitudinal direction: "Relaxation" of the spring 4 causes a longitudinal movement of its distal extremity and then a longitudinal movement of the shield 3 so as to proximally extend from the distal end of the tubular body 2 and then reach the activated position. In other embodiments not shown, the proximal extremity of the spring 4 may directly be secured to the tubular body 2.

In some embodiments, the syringe assembly 10 includes a first locking mechanism 31 for locking the safety shield 3 in the retracted position. Indeed, without said first locking mechanism 31, the shield 3 could not stay in the retracted position, because of the spring 4. In other words, said first locking mechanism 31 holds in place the shield 3 and/or the spring 4, and thus prevents the spring 4 from longitudinally moving the shield 3 towards the activated position. The force of the spring 4 is insufficient by itself to cause disengagement of the shield 3.

However, the first locking mechanism 31 is manually releasable by a user so as to let the safety shield 3 reach the activated position. In other words, the user can, by an action (see below) trigger the deactivation of the first locking mechanism 31 such that it stops restricting movement of the shield 3 and/or the spring 4. Thanks to the spring 4, the shield 3 switches from the retracted position to the activated position. We refer to as "activation of the shield" the manual release of the first locking mechanism 31, hence the word "activated" state of the shield 3 when extended.

The shield 3 is naturally held in the activated position by the spring 4, but a sufficient force could move the safety shield 3 back to the retracted position and then making apparent the needle 11. In some embodiments, the present syringe assembly advantageously comprises a second locking mechanism 32 for locking the safety shield 3 in the activated position after the first locking mechanism 31 is released. A difference with the first locking mechanism 31 is that the second locking mechanism 32 is not manually (even accidentally or unintentionally) releasable by a user (or at least significantly more difficult to release): the needle 11 cannot become exposed again, hence complete safety is guaranteed.

To sum up, the safety shield 3 is initially held by the first locking mechanism 31 in the retracted position, then, after medication delivery completion, the user releases it and the safety shield 3 longitudinally moves up to engage the second locking mechanism 32, which prevents it from switching back to the restricted position.

While locking mechanisms of the safety shield 3 are known, the present assembly distinguishes from the prior art in an astute architecture of the tubular body 2, the shield 3 and the locking mechanisms 31, 32, that is completely independent from the injection movement. For instance, while figure 2 represents the plunger rod 14 completely pulled down, i.e. the medication fully delivered, it is in practice possible to deploy the shield 3 regardless of the position of the plunger rod 14.

In particular, as depicted by figures 1 and 2:
- the tubular body 2 includes at least one longitudinal slot 21, i.e. a slit extending on the tubular body surface along said longitudinal axis of the barrel 12 and/or the tubular body 2. The longitudinal slot 21 is a linear slot.
- the safety shield 3 includes at least one a radial pin 30 engaging the longitudinal slot 21. In other words the pin 30 radially extends from the shield 3 through the tubular body 2, so that a user can handle the pin 30 as, the shield 3 is inside the tubular body, and is thus inaccessible to a user.

In some embodiments, the shield 3 and the pin 30 are integral, and therefore they move together.

The longitudinal slot 21 and the radial pin 30 are respectively located such that the radial pin 30 is at a proximal part 211 of the longitudinal slot 12 when the shield 3 is in the retracted state, and the radial pin 30 is at a distal part 212 of the longitudinal slot 12 when the shield 3 is in the activated state. Therefore, the radial pin 30 moves from the proximal part 211 of the longitudinal slot 12 to the distal part 212 of the longitudinal slot 21 when the safety shield 3 moves from the retracted position towards the activated position.

The first locking mechanism 31 is configured to hold the radial pin 30 at the proximal part 211 of the longitudinal slot 21.

Similarly, there is a second locking mechanism 32, which is configured to hold the radial pin 30 at the distal part 212 of the longitudinal slot 21.

The combination of the longitudinal slot 21 and the radial pin 30 allows simple yet very reliable locking mechanisms.

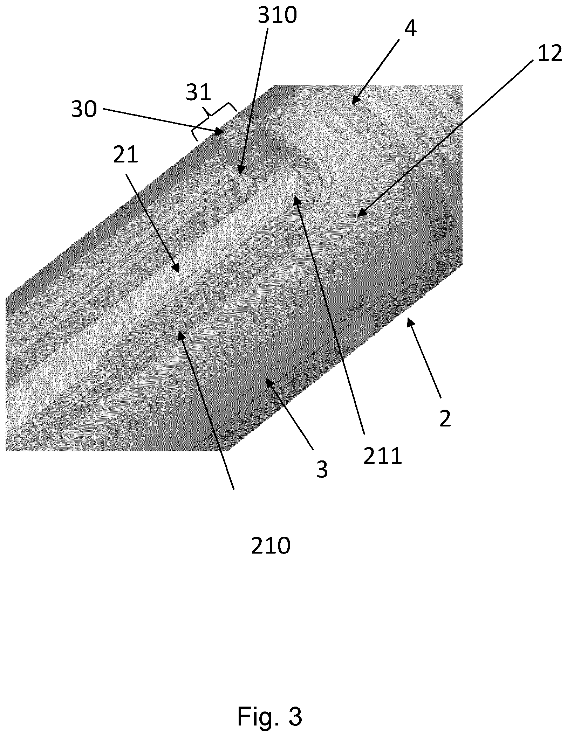

In some embodiments, the first locking mechanism 31 can be a bayonet mount with a notch 310 (see figure 3).

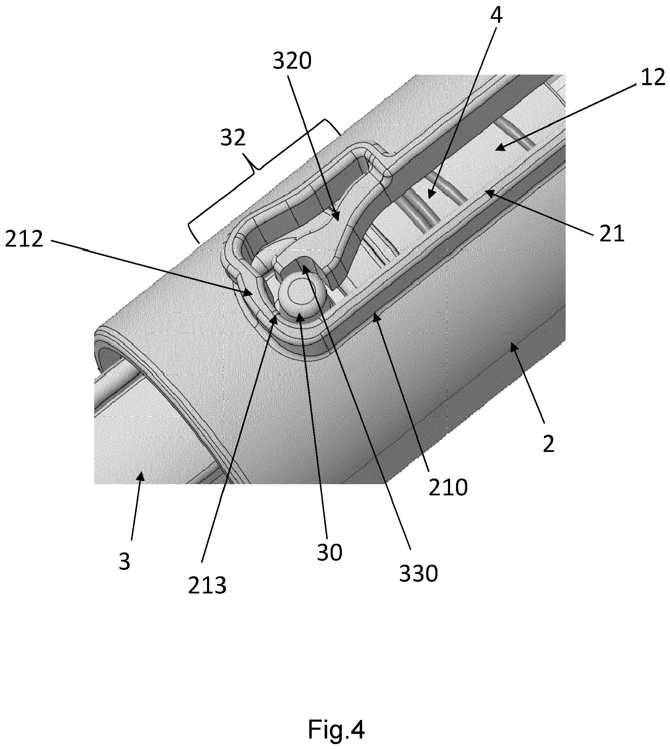

In some embodiments, the second locking mechanism 32 can be a semi-flexible fastener, preferably with a locking leg 320 (see figure 4). Said locking leg 320 preferably extends in cantilever from a wall of the longitudinal slot 32 with a slight angle (less than 45°) with respect to said longitudinal axis so as to come nearly into contact with the opposite wall of the longitudinal slot 32. To rephrase, the locking leg 320 extends forward with a slight lateral deviation, opening or closing a passage in the longitudinal slot 32. This allows the locking leg 320 to act as a "fishbone": the application of a longitudinal force in the distal direction deflects the leg 320 such that the passage in the longitudinal direction is opened, but the application of a longitudinal force in the rearward direction closes this passage. Consequently, the leg 320 may be forcedly deflected by the radial pin 30 only when the safety shield 3 moves from the retracted position towards the activated position. Preferably, as depicted by figure 4, the extremity of the locking leg 320 includes an angular cut 330 which is sized and configured to receive the radial pin 30 when the shield 3 is in the activated position for further improving the interlocking. At its distal end 212, the longitudinal slot 21 can include a curved portion 213, also configured and sized to receive the radial pin 30 when the shield 3 is in the activated position for further improving the interlocking. In other words, the edge of the cut 330 and/or of the curved portion 213 sensibly matches the shape of the radial pin 30.

As explained, the first locking mechanism 31 is manually releasable by the user, and/or the second locking mechanism 32 is not manually releasable by a user. This is allowed by configuring the longitudinal slot 21 such that the pin 30 radially protrudes from the tubular body 2 at the proximal part 211 of the longitudinal slot 12 (i.e. it can be moved by the user) and/or such that the pin 30 does not radially protrudes from the tubular body 2 at the distal part 212 of the longitudinal slot 12 (i.e. it cannot be reached and thus moved by the user).

To this end, the longitudinal slot 21 can be for instance provided with a rim 210 which radially protrudes from the tubular body 2. Said rim 210 encloses the distal part 212 of the longitudinal slot 21 but does not extend to the proximal part 211 of the longitudinal slot 21, as depicted by figures 3 and 4 (in this example, the rim 210 encloses all the longitudinal slot 21 except the proximal part 211). In some embodiments, the rim 210 is of variable height. In some embodiments, the rim 210 is sized so that the pin 30 does not protrude from the rim 210. In other words, when the pin 30 moves in the part of the slot 21 surrounded by the rim 210, the pin 30 is covered by the rim 210.

In figure 3, it can be seen that the radial pin 30 is locked in the notch 310 of the bayonet mount by the spring 4. The notch 310 is substantially orthogonal to the axis of the spring 4, thus preventing a longitudinal movement of the pin 30. This maintains the shield 3 in the retracted position. However the proximal part 211 of the longitudinal slot 12 is not enclosed by the rim 210, and thus the pin 30 protrudes from the tubular body 2: the user is able to push said pin 30 in an azimuthal direction (orthogonal to radial and longitudinal directions, causing rotation of the shield 3 along the longitudinal direction) out of the notch 310, up to being released. Thanks to the spring 4, a certain force is required to push the pin 30 out of the notch 310, preventing accidental release of the shield 3.

As it can see in figure 4, the radial pin 30 interlocks with the locking leg 320. The radial pin 30 sits against the curved portion 213 and the cut 330. The leg 320 can be forcedly deflected by the radial pin 30 when the safety shield 3 moves from the retracted position towards the activated position, in particular by the force of the spring 4, and when the pin 30 reaches the distal part 212 of the longitudinal slot 21 the leg 320 takes back its position and locks the pin 30. The leg 320 cannot be forcedly deformed by the radial pin 30 by an axial force in the opposite direction. This would only result in closing the passage and strengthening the lock. Nevertheless, the leg 320 could still theoretically be manually deflected by a user carefully applying a force in the correct azimuthal force while pulling rearward the pin 30 against the spring 4. However this is rendered difficult because the proximal part 211 of the longitudinal slot 12 is preferably enclosed by the rim 210, so that the pin 30 does not protrudes from the tubular body 2 and thus the user cannot reach the leg 320 or the pin 30. Therefore, the above-mentioned manipulations by a user are virtually impossible: the user cannot reach the leg 320 and the pin 30, hence the second locking mechanism 32 is not manually releasable by a user and once locked is largely non-reversible.

According to a second aspect, the invention is directed to the shielding system for an existing syringe 1 for use in medication delivery comprising a barrel 12 and a needle 11 at a distal end 122 of the barrel 12, and preferably a stopper 13 and a plunger rod 14 (said syringe 1 being preferably a pre-filled syringe), said shielding system comprising as explained:
- a tubular body 2 defining an enclosure, said barrel 12 extending at least partially within said enclosure;
- a safety shield 3 for the needle 11, longitudinally movable with respect to the tubular body 2 between a retracted position and an activated position;
- a spring 4 urging said safety shield 3 towards said activated position;
- a first locking mechanism 31 for locking the safety shield 3 in the retracted position, wherein the first locking mechanism 31 is manually releasable by a user so as to let the safety shield 3 reach the activated position;
- preferably a second locking mechanism 32 for locking the safety shield 3 in the activated position after the first locking mechanism 31 is released, wherein the second locking mechanism 32 is not manually releasable by a user.

Wherein:
- the tubular body 2 presents at least one longitudinal slot 21, and the safety shield 3 includes at least one radial pin 30 engaging the longitudinal slot 21 such that the radial pin 30 moves from a proximal part 211 of the longitudinal slot 12 to a distal part 212 of the longitudinal slot 21 when the safety shield 3 moves from the retracted position towards the activated position;
- the first locking mechanism 31 is configured to hold the radial pin 30 at the proximal part 211 of the longitudinal slot 21;
- preferably the second locking mechanism 31 is configured to hold the radial pin 30 at the proximal part 211 of the longitudinal slot 21.

A combination of the shielding system and said syringe 1 is a particular embodiment of a syringe assembly according to the first aspect.

According to a third aspect, the invention is directed to a method for operating the syringe assembly according to the first aspect (or the shielding system according to the second aspect), possibly after use of the syringe 1 for medication delivery.

Said method comprises an "activation" step of triggering release of the radial pin 30 by the first locking mechanism 31 (by instance by azimuthally pushing it) so that the spring 4 moves the safety shield 3 from the retracted position towards the activated position. The needle 11 is thus covered, the pin 30 is then preferably locked by the second locking mechanism 31 so that the shield 3 is kept in the activated position.

Not that in case of a shielding system for an existing syringe 1, said method may comprise a previous step of coupling the shielding system to said syringe 1, in particular by engaging the barrel 12 into the tubular body 2.

## Claims

1. A syringe assembly for use in medication delivery comprising:
- a syringe barrel (12) having a needle (11) at a distal end (122) of the barrel (12);
- a tubular body (2) defining an enclosure, said barrel (12) extending at least partially within said enclosure;
- a safety shield (3) for the needle (11), longitudinally movable with respect to the tubular body (2) between a retracted position and an activated position;
- a spring (4) urging said safety shield (3) towards said activated position;
- a first locking mechanism (31) for locking the safety shield (3) in the retracted position, wherein the first locking mechanism (31) is manually releasable by a user so as to let the safety shield (3) reach the activated position.
- the tubular body (2) including at least one longitudinal slot (21), and the safety shield (3) including at least radial pin (30) engaging the longitudinal slot (21) such that the radial pin (30) moves from a proximal part (211) of the longitudinal slot (12) to a distal part (212) of the longitudinal slot (21) when the safety shield (3) moves from the retracted position towards the activated position;
- the first locking mechanism (31) being configured to hold the radial pin (30) at the proximal part (211) of the longitudinal slot (21).

2. The syringe assembly of claim 1, comprising a second locking mechanism (32) for permanently and non-reversingly locking the safety shield (3) in the activated position after the first locking mechanism (31) is released.

3. The syringe assembly of claim 2, wherein the second locking mechanism (32) is configured to receive and then hold the radial pin (30) at the distal part (212) of the longitudinal slot (21).

4. The syringe assembly of any one of claims 2 to 3, wherein the second locking mechanism (32) comprises a locking leg (320) which is forcedly deflected by the radial pin (30) when the safety shield (3) moves from the retracted position towards the activated position.

5. The syringe assembly of any one of claims 2 to 4, wherein the pin (30) radially protrudes from the tubular body (2) when it is at the proximal part (211) of the longitudinal slot (12), and that the pin (30) does not radially protrudes from the tubular body (2) when it is at the distal part (212) of the longitudinal slot (12).

6. The syringe assembly of any of claims 4 to 5, wherein the longitudinal slot (21) is provided with a rim (210) enclosing the distal part (212) of the longitudinal slot (21) but not the proximal part (211) of the longitudinal slot (21).

7. The syringe assembly of any one of claims 4 to 6, wherein the locking leg (320) includes at its distal end a cut (330) configured and sized to receive the radial pin (30).

8. The syringe assembly of any one of claims 1 to 7, wherein the distal part (212) of the longitudinal slot (21) includes a curved portion (213) where the radial pin (30) sits when the safety shield (3) is its activated position.

9. The syringe assembly of any one of claim 1 to 8, wherein the needle (11) is covered by the safety shield (3) in the activated position and the needle (11) is not covered by the safety shield (3) in the retracted position.

10. The syringe assembly of any one of claims 1 to 9, wherein the first locking mechanism (31) is a bayonet mount.

11. The syringe assembly of any one of claims 1 to 10, comprising a prefilled syringe (1) comprising said barrel (12), said needle (11), a stopper (13) and a plunger rod (14).

12. A shielding system for a syringe (1) for use in medication delivery comprising a barrel (12) and a needle (11) at a distal end (122) of the barrel (12), said shielding system comprising:
- a tubular body (2) defining an enclosure, said barrel (12) extending at least partially within said enclosure;
- a safety shield (3) for the needle (11), longitudinally movable with respect to the tubular body (2) between a retracted position and an activated position;
- a spring (4) urging said safety shield (3) towards said activated position;
- a first locking mechanism (31) for locking the safety shield (3) in the retracted position, wherein the first locking mechanism (31) is manually releasable by a user so as to let the safety shield (3) reach the activated position;
- the tubular body (2) including a longitudinal slot (21), and the safety shield (3) including a radial pin (30) engaging the longitudinal slot (21) such that the radial pin (30) moves from a proximal part (211) of the longitudinal slot (12) to a distal part (212) of the longitudinal slot (21) when the safety shield (3) moves from the retracted position towards the activated position;
- the first locking mechanism (31) being configured to hold the radial pin (30) at the proximal part (211) of the longitudinal slot (21).

13. A method for operating a syringe assembly of any one of claims 1 to 10 or a shielding system of claim 11, comprising a step of triggering release of the radial pin (30) by the first locking mechanism (31) so that the spring (4) moves the safety shield (3) from the retracted position towards the activated position.
